# EUROPEAN PATENT APPLICATION

(11) **EP 2 468 312 A1**
(43) Date of publication of application: **27.06.2012**
(21) Application number: 10810047.0
(22) Date of filing: 16.08.2010
(51) Int. Cl.: A61L 27/00

(54) **SHEET FOR CORNEAL TRANSPLANTS**

(30) Priority: 19.08.2009 JP 2009190415
(71) Applicant: Tohoku University, Aoba-ku Sendai-shi Miyagi 980-8577 (JP); Kyoto University, Sakyo-ku Kyoto-shi Kyoto 606-8501 (JP)
(72) Inventor: NISHIDA, Kohji, Senda-shi Miyagi 980-8577 (JP); HAYASHI, Ryuhei, Sendai-shi Miyagi 980-8577 (JP); WATANABE, Ryo, Senda-shi Miyagi 980-8577 (JP); TABATA, Yasuhiko, Kyoto-shi Kyoto 606-8507 (JP)
(74) Representative: HOFFMANN EITLE
(86) International application number: PCT/JP2010/064125
(87) International publication number: WO 2011/021706

(57) **Abstract**

The present invention relates to a sheet for corneal transplants comprising corneal endothelial cells on a gelatin hydrogel, which is obtainable by seeding and culturing corneal endothelial cells on a gelatin hydrogel coated with collagen. The sheet of the present invention is extremely useful as a sheet for corneal transplants not only for its biocompatibility and biodegradability, but also for its high transparency.

## Description

### Technical Field

The present invention relates to a sheet for corneal transplants having a gelatin hydrogel as a carrier. In more detail, the present invention relates to a highly transparent sheet for corneal transplants prepared by culturing corneal endothelial cells on a gelatin hydrogel.

### Background Art

The cornea is composed of five layers, which are, from the outside, corneal epithelial layer, Bowman's layer, corneal stromal layer, Descemet's membrane, and corneal endothelial layer. Among them, the innermost corneal endothelial layer is a single layer of cells, and this layer of cells is responsible for taking up necessary materials for the cornea from hydatoid, while discharging water in the cornea into hydatoid to maintain constant corneal thickness and to keep the cornea transparent. Reduction in the number of corneal endothelial cells leads to insufficient water discharge, causing corneal endothelial disease such as corneal opacity and bullous keratopathy. Human corneal endothelial cells do not proliferate in vivo; therefore, once damaged, transplantation is the only option for radical treatment.

Conventionally, penetrating keratoplasty has been performed for corneal endothelial disease; however, there are problems of absolute shortage of donors and post-transplant rejection reaction. In response to these problems, Descemet Stripping Endothelial Keratoplasty (DSEK), which is a method involving acquiring the corneal endothelium (partially containing the corneal stroma) from the imported research human cornea from U.S. eye bank and transplanting it into an affected eye, is known. Because DSEK transplants corneal endothelial cells, it will reduce the rejection reaction compared to penetrating keratoplasty. However, this method cannot be a radical solution to the problems of rejection reaction and donor shortage.

Meanwhile, although still in the research phase, a therapeutic method using the patient's own corneal endothelial cells is also being developed. Since this method enables acquisition of a large amount of corneal endothelial cells by propagating the corneal limbal cells of the healthy eye, it could solve the aforementioned problem of donor shortage. However, normally, cultured corneal endothelial cells form a monolayer and the intercellular connection is not very strong; therefore, direct use of the cultured corneal endothelial cells for transplantation is difficult in terms of operability and strength. Also, although cultured cells are normally detached and collected from culture containers by treatment with an enzyme such as trypsin or with chemicals, there are problems that such treatment would disrupt the desmosome structure, causing drastic reduction in the engraftment, strength, and function of the transplanted cell sheet.

In response to the aforementioned problems, the present inventors have developed a method of transplanting a sheet of cultured corneal endothelial cells obtained by using a culture dish having bound thereto a temperature responsive polymer, the hydration force of which varies within a temperature range of 0 to 80°C. This method enables acquisition of a cell sheet only by lowering temperature without involving enzymes, and experimental transplantation into the rabbit cornea was successful (Patent Literature 1 and Non Patent Literature 1) . However, because no carrier is used in this method, handling of the harvested cultured corneal endothelial cells is difficult, posing a problem for practical clinical application.

Also, although still in the animal experiment phase, a method using atelocollagen as a carrier for transplantation of cultured corneal endothelial cells is also known (Non Patent Literature 2). However, atelocollagen has poor bioadhesive and biodegradable properties, and there is a report that it exfoliates from the endothelial surface after transplantation and remains in the anterior chamber. Thus, there is a concern that this might stimulate endothelial cells, consequently causing a reduction in the cell density. Further, a method including mounting cultured corneal endothelial cells on a cylindrically-formed gelatin disk and transplanting the whole disk is also reported (Non Patent Literature 3). However, the gelatin disk is as thick as about 800 µm and requires a long time for biodegradation, and also, there is a concern that degradation might induce inflammation.

In the tissue engineering in regenerative medicine, a method including culturing cells and tissues on a biodegradable culture carrier and transplanting them with the carrier into the affected part is known. The use of a water soluble polymer such as polyvinyl alcohol and a hydrogel such as collagen as a constituent material of the carrier is also known (Patent Literature 2). A gelatin hydrogel is a hydrogel obtained by cross-linking the gelatin molecules by thermal reaction and the like, and it has not only high bioabsorbability and biocompatibility but also an excellent sustained-release effect. For this, the use of a gelatin hydrogel as a sustained-release substrate for cytokines such as bFGF and BMP and for poorly water-soluble drugs is reported (Patent Literatures 3 to 5, and the like). However, the use of a gelatin hydrogel in corneal cell culture as a carrier for corneal endothelial transplantation has been unknown to date.

### Citation List

### Patent Literature

Patent Literature 1: WO2004/073761
Patent Literature 2: Japanese Patent Laid-Open No. 2002-186847
Patent Literature 3: Japanese Patent Laid-Open No. 2004-203829
Patent Literature 4: Japanese Patent Laid-Open No. 2007-332106
Patent Literature 5: Japanese Patent Laid-Open No. 2008-137975

### Non Patent Literature

Non Patent Literature 1: Ide T, et al., Biomaterials, 2006 Feb., 27 (4): 607 to 614
Non Patent Literature 2: Koizumi N., et. al., Invest. Ophthalmol Vis. Sci., 2007 Oct., 48 (10): 4519 to 4526
Non Patent Literature 3: Hsiue GH et al., Transplantation. 2006 Feb., 15; 81 (3): 473 to 476

### Summary of Invention

### Technical Problem

A substrate for transplantation used for corneal transplants is required to be not only biocompatible and biodegradable but also transparent. An object of the present invention is to determine the best substrate for corneal endothelial transplantation satisfying the above conditions and provide, using this substrate, a sheet for corneal transplants that can be directly applied to the affected part.

### Solution to Problem

The present inventors searched for a material satisfying the following conditions as a substrate for transplantation used for corneal transplants, and selected a gelatin hydrogel.
1) Capable of transplanting cultured corneal endothelial cells without impairing the function and form of the endothelial cells in the process of transplantation.
2) Having favorable engraftment into the stroma so as not to cause exfoliation of the sheet, or being rapidly biodegradable so as not to cause exfoliation of the sheet into the anterior chamber, thereby not damaging the transplanted corneal endothelium.
3) Having favorable transparency and biocompatibility after transplantation, causing no inflammation reaction and the like, and keeping the cornea transparent.

The present inventors then processed gelatin into a sheet and subjected it to cross-linking treatment to produce a gelatin hydrogel sheet, and subsequently, using this sheet as a carrier, cultured corneal endothelial cells. Then, they transplanted the resulting sheet into the anterior segment of a mouse model of bullous keratopathy and evaluated the degradability and therapeutic effect of the sheet. As a result, it was confirmed that the sheet for corneal transplants having a gelatin hydrogel as a carrier showed high engraftment and transparency of cells and exhibited an excellent post-transplantation course.
That is, the present invention relates to a sheet for corneal transplants comprising corneal endothelial cells on a gelatin hydrogel.

The thickness of the gelatin hydrogel in the sheet for corneal transplants of the present invention is preferably 10 to 200 µm, more preferably 30 to 100 µm.

Also, the percentage water content of the gelatin hydrogel is preferably 92 to 99%, more preferably 95 to 98%. Further, the corneal endothelial cells used are ZO-1 or Na⁺/K⁺-ATPase positive cells.

The gelatin hydrogel used in the present invention is produced by subjecting gelatin to thermal cross-linking treatment. Although the gelatin hydrogel is degraded in vivo after transplantation, the rate of degradation can be adjusted by the degree of cross-linking and the like. That is, it may be possible to adjust the rate of degradation to such a rate that the gelatin hydrogel disappears by degradation in vivo within one to six months after transplantation, or to such a rate that the gelatin hydrogel is engrafted into the stromal tissue without being biodegraded for more than one year.
The corneal endothelial cells used in the present invention are preferably prepared from cells derived from the patient.

The sheet for corneal transplants of the present invention is obtained by seeding and culturing corneal endothelial cells on a gelatin hydrogel sheet coated with collagen.

Here, the collagen coating is performed not by a conventional method involving immersing a sheet into a collagen solution but by a method involving applying a collagen solution over the sheet surface. The cell adherence property and barrier function of the sheet are remarkably improved by coating collagen by this method. No particular limitation is imposed on the temperature, humidity, and time of the coating treatment. Also, similarly, no limitation is imposed on the concentration and kind of the collagen solution.

Specifically, the sheet for corneal transplants is obtained by seeding and culturing corneal endothelial cells on a gelatin hydrogel sheet coated with collagen and detaching the gelatin hydrogel sheet with the corneal endothelial cells from a culture container on or after the seventh day after the corneal endothelial cells become confluent. As described above, the density of cells on the gelatin hydrogel in the obtained sheet for corneal transplants is 2000 cells/mm² or more, preferably 2500 cells/mm² or more, and more preferably 3000 cells/mm² or more.

### Advantageous Effects of Invention

Because the cultured corneal endothelial cells form a monolayer, handling of the cells during transplantation is difficult. Thus, a strong, transparent carrier is needed to achieve stable transplantation results. The gelatin hydrogel used in the present invention has appropriate strength and excellent transparency, and when used as a carrier for transplantation, it will remarkably improve the strength and operability of the sheet for corneal transplants. Also, because the gelatin hydrogel is degraded in vivo and then disappears, there is no concern that this gelatin hydrogel may cause damage to the corneal endothelium, and further, the corneal transparency is recovered in the early post-transplantation period. Further, because gelatin hydrogels have been already used as a clinical material, the sheet for corneal transplants of the present invention using a gelatin hydrogel can be safely used for clinical application.

### Brief Description of Drawings

[Figure 1] Figure 1 shows a photographic image comparing the transparency between the gelatin hydrogel sheet and the atelocollagen sheet.
[Figure 2] Figure 2 shows the evaluation results of the mechanical characteristics of the gelatin hydrogel sheet and the atelocollagen sheet (A: strength of each sheet in a tensile test (48 hr, 24 hr, 12 hr, atelocollagen, and 6 hr, from the left in the graph), B: strength of each sheet in a tensile test, C: A break point of each sheet in a tensile test, and D: An elastic modulus of each sheet)).

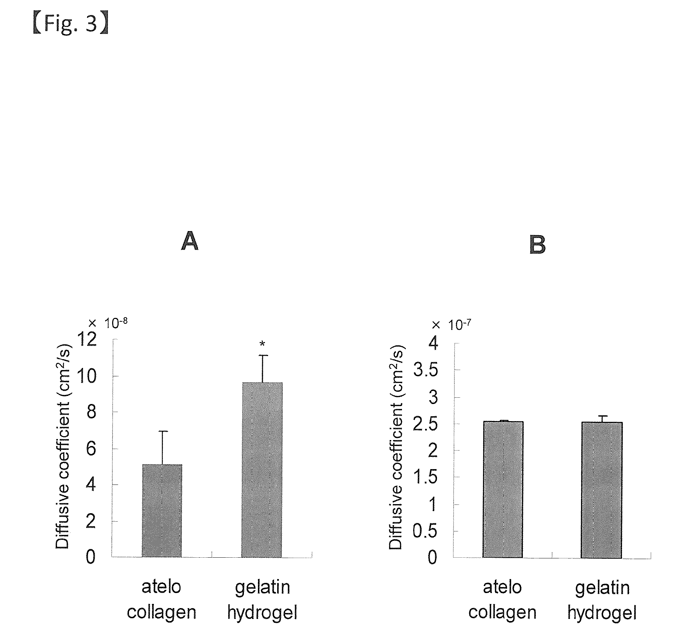

[Figure 3] Figure 3 shows the results of a permeability test of atelocollagen and the gelatin hydrogel sheet (A: albumin, B: glucose).
[Figure 4] Figure 4 shows the evaluation results of cell adherence property (phase-contrast microscopic image) and improvement in barrier function (expression of ZO-1) by different coating methods applied to the gelatin hydrogel sheet on which corneal endothelial cells are cultured ((a) a phase-contrast microscopic image (Day 1), (b) ZO-1 immunofluorescent staining (Day 10); A: sheets immersed in a 0.15 mg/ml collagen solution (conventional method), B: sheets onto which a 3.0 mg/ml collagen solution was applied, and C: atelocollagen (comparative control), from the left).
[Figure 5] Figure 5 shows the evaluation results of the transparency of the gelatin hydrogel sheet on which corneal endothelial cells are cultured (left: atelocollagen, right: gelatin hydrogel sheet).
[Figure 6] Figure 6 shows the evaluation of the corneal endothelial cells cultured on the gelatin hydrogel sheet (A: analysis of Na⁺/K⁺-ATPase and ZO-1 expression (immunostaining images), B: an HE staining image, and C: a scanning microscopic image, each scale indicates 200 µm).
[Figure 7] Figure 7 shows HE staining images of the gelatin hydrogel sheet (a) before transplantation and (b) after transplantation into the rabbit anterior chamber (each scale indicates 200 µm).
[Figure 8] Figure 8 shows the results of comparison of the thickness of the cultured corneal endothelial cell sheets prepared with the gelatin hydrogel sheet in the transplanted, non-transplanted, and normal eyes in rabbit models of bullous keratopathy as of (a) Day 7 after transplantation and (b) Day 21 after transplantation.
[Figure 9] Figure 9 shows (A) HE staining images and (B) DiI staining images of the cultured corneal endothelial cell sheets prepared with the gelatin hydrogel sheet in rabbit model of bullous keratopathy as of 21 day after transplantation. The images on the right are the magnified images of the boxed area in the images on the left.

The present specification encompasses the contents described in the specification of Japanese Patent Application No. 2009-190415, based on which the present application claims priority.

### Description of Embodiments

### 1. Gelatin hydrogel

### 1.1 About the gelatin hydrogel

The "gelatin hydrogel" of the present invention is a hydrogel obtained by cross-linking the gelatin molecules by subjecting gelatin to chemical reactions or thermal dehydration treatment, or by irradiating gelatin with radiation, ultraviolet ray, electron beam, or the like.

At this point, the term "gelatin" refers to a modified collagen that is irreversibly converted into a water-soluble protein as a result of cleavage of the salt and hydrogen bonds between the peptide chains in the collagen by acid treatment, alkali treatment, enzyme treatment, and the like.

The gelatin used in the present invention may be either acidic gelatin or basic gelatin. In the present specification, the term "acidic gelatin" means a gelatin having an isoelectric point of less than 7.0 and 2.0 or more, preferably 6.5 or less and 4.0 or more, and more preferably 5.5 or less and 4.5 or more, after preparing collagen with alkali treatment. Also, the term "basic gelatin" means a gelatin having an isoelectric point of 7.0 or more and 13.0 or less, preferably 7.5 or more and 10.0 or less, and more preferably 8.5 or more and 9.5 or less, after preparing collagen with acid treatment. For example, as the "acidic gelatin", a reagent with an isoelectric point (IEP) of 5.0 supplied by Nitta Gelatin, Inc. and the like, and as the basic gelatin, similarly, a reagent with IEP of 9.0 supplied by Nitta Gelatin, Inc. and the like can be used.

Either one of these gelatins is appropriately selected for use according to the medicinal components to be incorporated and the purpose of use. For example, because bFGF has an IEP of 4.6, an acidic gelatin is used when such a medicinal agent is incorporated, whereas when a protein medicinal agent having an IEP of 7 or less is incorporated, a basic gelatin is used.

### 1.2 Cross-linking of gelatin

The degree of cross-linking of gelatin can be appropriately selected according to desired levels of biodegradability, percentage water content, and bioabsorbability. The cross-linking may cross-link any part of collagen constituting gelatin; however, it is particularly preferable to cross-link a carboxyl group and a hydroxyl group, a carboxyl group and a ε-amino group, and ε-amino groups to each other. By introducing cross-linking as shown above, the resulting complex will attain desired mechanical strength characteristics. Also, depending on the rate of introduction of cross-linking, the rate of degradation in vivo (duration in the body) can also be controlled. Generally in the hydrogel, as the concentrations of gelatin and cross-linking agents as well as the time of cross-linking are increased, the degree of cross-linking is increased, while bioabsorbability is lowered.

The cross-linking of gelatin can be performed by a method such as thermal reaction (such as thermal dehydration treatment), cross-linking by chemical technique using cross-linking agents and condensing agents, and cross-linking by physical technique using γ-ray, ultraviolet ray, electron beam, and the like. When performing chemical cross-linking, examples of the cross-linking agent used include aldehyde cross-linking agents such as glutaraldehyde and formaldehyde; isocyanate cross-linking agents such as hexamethylene diisocyanate; carbodiimide cross-linking agents such as 1-ethyl-3-(3-dimethylaminopropyl) carbodiimide hydrochloride; polyepoxy cross-linking agents such as ethylene glycol diethyl ether; and transglutaminase. The amount of the cross-linking agent added is appropriately determined depending on the cross-linking agent used. The degree of cross-linking can be appropriately selected according to the desired percentage water content, namely the level of bioabsorbability of the hydrogel. When gelatin is used as a bioabsorbable polymer, the concentrations of gelatin and cross-linking agents for preparation of a hydrogel preferably range from 1 to 20 w/w% for gelatin and from 0.01 to 1 w/w% for cross-linking agents. Although no particular limitation is imposed on the conditions of crosslinking reaction, for example, the cross-linking reaction can be performed at 0 to 40°C, preferably at 25 to 30°C for 1 to 48 hours, preferably for 12 to 24 hours. Generally in the hydrogel, as the concentrations of gelatin and cross-linking agents as well as the time of cross-linking are increased, the degree of cross-linking is increased, while bioabsorbability is lowered. The percentage water content and the amount of cross-linking agents are described in Japanese Patent Laid-Open No. 2004-203829.

Although cross-linking may be performed by any method, thermal cross-linking (such as thermal dehydration treatment) is preferably performed in the present invention. This is because when chemical cross-linking is performed, cross-linking agents may remain in the hydrogel, and also, under certain conditions, cross-link may be formed only on the hydrogel surface, failing to achieve uniform cross-linking. In contrast, when thermal cross-linking is performed, cross-link is uniformly formed over the entire gelatin hydrogel and the desired biodegradability is achieved.

When cross-link is formed by thermal reaction, specifically, the reaction is carried out under the conditions of 140°C to 160°C for six to 72 hours in the vacuum state. Under such condition settings, the rate of degradation of a gelatin hydrogel in vivo (duration in the body) can be controlled. In Examples of the present invention, a gelatin hydrogel prepared by heating at 160°C at 0.01 Torr for 72 hours in vacuum was used.

### 1.3 Biodegradability

Degradability of the gelatin hydrogel can be adjusted by the aforementioned degree of cross-lining and thickness of the sheet. Degradability can be controlled in a range of 14 days to one year or more within the animal's body, for example, in the anterior segment. In the present invention, if the gelatin hydrogel is degraded too fast, it cannot fulfill its role as a scaffold for corneal endothelial cells, resulting in poor engraftment of corneal endothelial cells. In view of the above, degradability is set preferably at 14 days to six months, particularly preferably at about one to three months. Alternatively, if the gelatin hydrogel is well engrafted into the stroma without exfoliation, it is also possible to set the time to degradation at one year or more, i.e., such a period of time that biodegradation of the hydrogel virtually does not occur.

The degree of cross-linking of gelatin can be evaluated by using the percentage water content as an index. The percentage water content is the weight percent of water in a hydrogel relative to the weight of a swollen hydrogel. When the percentage water content is large, the degree of cross-linking of the hydrogel becomes low, making the hydrogel susceptible to degradation. The percentage water content for exhibiting preferable degradability is about 92 to 99 w/w%, more preferably about 95 to 98 w/w%.

### 1.4 Form of the gelatin hydrogel

The gelatin hydrogel sheet of the present invention can be shaped in any form; however, it is shaped into a sheet for application to a carrier for transplantation of cultured corneal endothelial cells in the present invention. In consideration of application to corneal endothelial transplantation, the thickness of the sheet is preferably about 10 to 200 µm, more preferably about 30 to 100 µm.

### 1.5 Transparency of the gelatin hydrogel

The gelatin hydrogel of the present invention has excellent transparency. Compared to a conventional carrier such as atelocollagen, the transparency of the gelatin hydrogel of the present invention is remarkably higher. Also, the gelatin hydrogel can maintain its transparency after being applied to the affected part.

### 2. Corneal endothelial cell

### 2.1 Donor- or patient-derived corneal endothelial cell

The corneal endothelial cells used in the present invention collectively refer to the cells contained in the corneal endothelial cell layer, including corneal endothelial stem cells and corneal endothelial progenitor cells. Also, as the corneal endothelial cells, the corneal endothelium obtained from a donor other than the patient, for example from the imported research human cornea from U.S. eye bank, as well as the corneal endothelial cells derived from a patient undergoing transplantation may be used. From the viewpoint of prevention of rejection reaction, the use of the patient-derived cells is desirable. However, given that corneal endothelial cells do not proliferate in vivo as described above, acquisition of corneal endothelial cells from the healthy eye of the patient is technically possible but realistically difficult.

### 2.2 Corneal endothelial cell differentiated from stem cell

As the corneal endothelial cells, corneal endothelial stem cells, tissue stem cells other than corneal endothelial stem cells, and embryonic stem cells (ES cells) may be used, or corneal endothelial cells induced from induced pluripotent stem cells may also be used. Although no particular limitation is imposed on the preparation method of corneal endothelial stem cells and induced pluripotent stem cells used, the cells are preferably derived from the patient in need of treatment.

The induced pluripotent stem cell refers to a mammalian somatic cell or an undifferentiated stem cell that is reprogrammed (initialized) so as to attain similar pluripotency to ES cells by introduction of specific factors. The "induced pluripotent stem cell (iPS cell)" was so-named and first established by Yamanaka et al. by transfecting mouse fibroblasts with four factors, namely Oct3/4, Sox2, Klf4, and c-Myc (Takahashi K, Yamanaka S., Cell, (2006) 126: 663 to 676). In addition to this iPS cell, a human iPS cell established by transfecting human fibroblasts with the same four factors (Takahashi K, Yamanaka S., et al. Cell, (2007) 131: 861 to 872), and further, an iPS cell produced by a method not involving c-Myc (Nakagawa M, Yamanaka S., et al. Nature Biotechnology, (2008) 26, 101 to 106) may also be used.

Also, induced pluripotent stem cells produced by Thomson et al. in University of Wisconsin by transfecting human fibroblasts with four genes, namely OCT3/4, SOX2, NANOG, and LIN28 (Yu J., Thomson JA. et al., Science (2007) 318: 1917 to 1920), induced pluripotent stem cells produced by Daley et al. in Harvard University by transfecting skin cells with six genes, namely OCT3/4, SOX2, KLF4, C-MYC, hTERT, and SV40 large T (Park IH, Daley GQ. et al., Nature (2007) 451: 141 to 146), and induced pluripotent stem cells produced by Sakurada et al., by using undifferentiated stem cells present in tissues after birth as a cell source and transfecting these cells with Oct3/4, Sox2, Klf4, c-Myc, and the like (Japanese Patent Laid-Open No. 2008-307007) may also be used.

In addition, induced pluripotent stem cells produced by transfecting mouse neural progenitor cells and the like with OCT3/4, KLF4, and low molecular weight compounds (Shi Y., Ding S., et al., Cell Stem Cell, (2008) Vol 3, Issue 5, 568 to 574), induced pluripotent stem cells produced by transfecting mouse neural stem cells endogenously expressing SOX2 and C-MYC with OCT3/4 and KLF4 (Kim JB., Scholer HR., et al., Nature, (2008) 454, 646 to 650), induced pluripotent stem cells produced by using the Dnmt and HDAC inhibitors without using C-MYC (Huangfu D., Melton, DA., et al., Nature Biotechnology, (2008) 26, No 7, 795 to 797), or any of the induced pluripotent stem cells described in all of the published patents: Japanese Patent Laid-Open No. 2008-307007, Japanese Patent Laid-Open No. 2008-283972, US2008-2336610, US2009-047263, W02007-069666, WO2008-118220, W02008-124133, WO2008-151058, WO2009-006930, WO2009-006997, and WO2009-007852 may also be used.

Induction of differentiation of tissue stem cells, ES cells, and induced pluripotent stem cells into corneal endothelial cells may be direct or indirect via neural crest cells and corneal stromal stem cells. That is, tissue stem cells, ES cells, and induced pluripotent stem cells are once differentiated into neural crest cells or corneal stromal stem cells, and then the resulting neural crest cells and corneal stromal stem cells are further differentiated into corneal endothelial cells by TGFb2 and the like in accordance with a publicly known technique (Japanese Patent Application No. 2008-123562).

As a method for inducing neural crest cells from ES cells, the Stromal Cell-derived Inducing Activity (SDIA) method reported by Sasai and Mizuseki et al. is known. The SDIA method is a method for inducing differentiation of ES cells into neural crest cells by using mouse-derived stromal cells (PA6 cells) as feeder cells (Kawasaki, H., Sasai, Y. et al. (2000) Neuron 28, 31 to 40., Kawasaki, H., Sasai, Y. et al. (2002) Proc. Natl. Acad. Sci. USA 99, 1580 to 1585, and Mizuseki, K., Sasai, Y. et al. (2003) Proc. Natl. Acad. Sci. USA 100, 5828 to 5833). In addition, as a method for inducing neural crest cells from ES cells, a method using ST2 cells (Motohashi T et al. Stem Cells, 2007; 25: 402 to 410) and a method using MS-5 cells (Lee G et al. Nat Biotechnol. 2007; 25: 1468 to 1475) are also known.

Also, as a method for inducing neural crest cells from ES cells and induced pluripotent stem cells, a method of differentiation induction utilizing modified SFEB method proposed by the present inventors (submitted) is also possible. The Serum-Free Floating Culture of Embryoid Body-like Aggregates (SFEB) method is a method for inducing nerve cells from mouse ES cells by combining a specific serum free culture liquid and floating aggregate culture in the absence of feeder cells (Watanabe K et al., Nature Neuroscience 2005; 8: 288 to 296).

### 3. Corneal endothelial cell culture on the gelatin hydrogel

The sheet for corneal transplants of the present invention is prepared by coating the aforementioned gelatin hydrogel sheet with collagen (type I or type IV) and seeding and culturing corneal endothelial cells on the collagen-coated sheet.

### 3.1 Collagen coating

Collagen coating of the gelatin hydrogel is performed with an aim to improve cell adherence property and corneal endothelial function. Type I or type IV collagen is preferably used, and it is preferably atelocollagen devoid of antigenicity. Collagen coating is performed in accordance with a routine method, namely by diluting collagen with diluted hydrochloric acid (pH 3.0) 10-fold and thinly applying the diluted collagen over a gelatin hydrogel, and then letting it dry. The coated sheet is preferably washed with Phosphate-Buffered Salines (PBS) (Invitrogen) before use.

Cell adhesive proteins, peptides, glycoproteins, and the like such as laminins, serum, and FNC coating Mix (Athena Enzyme Systems) may also be used alone or a mixture of these substances may also be used instead of collagen. The concentration of coating is 10⁻⁴ to 10⁰ mg/ml, and an aqueous solution having any kind of composition can be used.

Here, collagen coating is performed not by a conventional method of immersing a sheet into a collagen solution but by a method of applying (smearing) a collagen solution over the sheet surface. The cell adherence property and barrier function of the sheet are remarkably improved by coating by this method. In the coated sheet, coated collagen is immobilized near the surface of the gelatin sheet by drying. This immobilization to the surface is important for the adhesion and manifestation of functions of the cells. Because it is speculated that cell adhesion and endothelial functions would be reduced in the absence of coating, it is desirable to perform coating.

### 3.2 Medium

Culture on the gelatin hydrogel sheet can be performed using a medium ordinarily employed for adherent cell culture. Any medium that can be used for animal cell culture such as DMEM medium, BME medium, α MEM medium, Dulbecco MEM medium, BGJb medium, CMRL 1066 medium, Glasgow MEM medium, Improved MEM Zinc Option medium, IMDM medium, Medium 199, Eagle MEM medium, Ham's medium, RPMI 1640 medium, Fischer's medium, McCoy's medium, William's medium E, and a mixed medium of these media can be used.

Using these media as basic media, various nutrient sources necessary for maintenance and proliferation of corneal endothelial cells and each component necessary for induction of differentiation of corneal endothelial cells may be appropriately added.

For example, the medium can contain, as the nutrient source, serum, basic fibroblast growth factor (bFGF), epithelial growth factor (EGF), carbon sources such as glycerol, glucose, fructose, sucrose, lactose, honey, starch, and dextrin, also, hydrocarbons such as fatty acid, oil and fat, lecithin, and alcohols, nitrogen sources such as ammonium sulfate, ammonium nitrate, ammonium chloride, urea, and sodium nitrate, inorganic salts such as table salt, potassium salt, phosphate, magnesium salt, calcium salt, iron salt, and manganese salt, potassium dihydrogen phosphate, dipotassium hydrogen phosphate, magnesium sulfate, sodium chloride, ferrous sulfate, sodium molybdate, sodium tungstate, and manganese sulfate, various vitamins, amino acids, and the like.

In addition to the above, amino acid reducing agents such as pyruvic acid and β mercaptoethanol, serum alternatives, and the like can be added as needed. It should be noted that examples of the serum alternative include albumin (such as lipid-rich albumin), transferrin, fatty acid, insulin, collagen precursors, trace elements, β mercaptoethanol or 3' thiolglycerol, commercially available Knockout Serum Replacement (KSR), Chemically-defined Lipid concentrated (manufactured by Gibco), and Glutamax (manufactured by Gibco).

The medium obtained by blending the aforementioned components has a pH ranging from 5.5 to 9.0, preferably from 6.0 to 8.0, and more preferably from 6.5 to 7.5.

### 3.3 Culture conditions

Corneal endothelial cells are seeded in the gelatin hydrogel containing the aforementioned medium at a seeding density of 500 to 4500 cells/mm², preferably 1500 to 3500 cells/mm², and cultured under the conditions of 36°C to 38°C, preferably 36.5°C to 37.5°C and 1% to 25% O₂ and 1% to 15% CO₂. Culture days are at least three days or more, preferably seven days or more after the cells become confluent. The density of cells on the gelatin hydrogel obtained at last is 2000 cells/mm² or more, preferably 2500 cells/mm² or more, and more preferably 3000 cells/mm² or more.

### 4. Sheet for corneal transplant

As the sheet for corneal transplants of the present invention uses a gelatin hydrogel as a carrier, it is capable of markedly improving the strength and operability of the corneal endothelial cell layer, which is a single layer only about 10 µm thick by itself. The corneal endothelial cells on the sheet express the tight junction protein ZO-1, which exhibits barrier functions, and are adhered well to the sheet. Because the sheet can be detached from the culture container without application of treatment with enzymes such as trypsin and Dispase, the desmosome structure of the cell as well as the inherent structure and strength of the corneal endothelial cell layer are maintained.

Also, because the gelatin hydrogel is highly transparent, the sheet for corneal transplants of the present invention has remarkably higher transparency than does a conventional publicly known sheet for corneal transplants having atelocollagen as a carrier, and the transparency of the sheet for corneal transplants of the present invention is maintained after transplantation as well.

The sheet for corneal transplants of the present invention can contain medicinal components as needed. Examples of such medicinal components include antitumor agents, antimicrobial agent, anti-inflammatory agents, anti-virus agents, anti-AIDS agents, low molecular weight medicines such as hormones, physiologically active peptides including osteogenic factors or bone growth factors, proteins, glycoproteins, polysaccharides, and nucleic acids. These medicinal components may be naturally derived or synthetically produced substances.

Specific examples of the medicinal component include a cell growth factor such as epithelial growth factor (EGF), fibroblast growth factor (FGF), platelet-derived growth factor (PDGF), hepatocyte growth factor (HGF), a transforming growth factor (TGF), and insulin-like growth factor (IGF), and, particularly with regard to osteoanagenesis, bone morphogenetic protein (BMP) such as BMP-2, BMP-4, BMP-5, BMP-6, BMP-7 (OP-1), and BMP-8(OP-2), glial cell line-derived neurotrophic factor (GDNF), neurotrophic factor (NF), platelet-rich plasma (PRP) containing numerous cell growth factors widely used in clinical dentistry, an anticancer agent such as interferon, interleukin-2, and ifosfamide, an antibiotic such as streptomycin, gentamicin, and gatifloxacin, a hypocholesterolemic agent such as atorvastatin, pravastatin, and simvastatin, lidocaine, protamine sulfate, sodium iodohippurate, iodinated sulfobromophthalein, heparin sodium, glucose, norepinephrine, dextran, thiopental sodium, sodium chromate injection, xylitol, procaine hydrochloride, tetracaine hydrochloride, tubocurarine chloride, suxamethonium chloride, amorphous insulin zinc, amyl nitrite, and ajmaline.

The gelatin hydrogel may be impregnated with the aforementioned medicinal components before or during culturing corneal endothelial cells thereon, or before or after preparing the sheet for transplants. The blending ratio of the medicinal component relative to the gelatin hydrogel is preferably about 5 times or less, further preferably about 5 to about 1/10⁴ times the mole of the gelatin hydrogel. Normally, the impregnation operation is completed at 4 to 37°C within 15 minutes to one hour, preferably at 4 to 25°C within 15 to 30 minutes, during which time the gelatin hydrogel is swollen in a solution containing medicinal components, and the medicinal components are conjugated with the gelatin hydrogel by physicochemical interactions to form a complex and are immobilized in the gelatin hydrogel. It is speculated that not only physical interaction such as Coulomb's force, hydrogen bonding strength, and hydrophobic interaction but also a coordinate bond between the functional group or metal of a medicine and the functional group of the hydrogel, and the like is involved in the binding between the medicinal components and the gelatin hydrogel either alone or in a combined manner.

The medicinal components are gradually released outside the sheet as the gelatin hydrogel is degraded in vivo and the gelatin molecules are solubilized in water. The rate of release is determined by the degree of degradation and absorption of the gelatin hydrogel used in vivo and by the degree of strength and stability of binding between the medicinal components and the gelatin hydrogel in a complex. The degree of degradation and absorption of the gelatin hydrogel in vivo can be adjusted by regulating the degree of cross-linking during preparation of the hydrogel.

In the present invention, for example when a negatively-charged substance such as a nucleic acid is used as the medicinal component, the gelatin hydrogel is preferably positively charged so that the medicinal component and the gelatin hydrogel form a stable complex. A stable gelatin hydrogel complex is formed by a strong bond (ionic bond) between the negative charge of the medicinal component and the positive charge of the gelatin hydrogel. The gelatin hydrogel can be cationized to be positively charged by introducing thereinto an amino group and the like in advance. By doing so, the bonding strength between the gelatin hydrogel and the medicinal component is increased and a more stable gelatin hydrogel complex can be formed. Meanwhile, when the medicinal component is a positively charged substance, the medicinal component and the gelatin hydrogel form a stable complex and the gelatin is anionized by interaction. As a result, the medicinal component interacts with the gelatin and is stably conjugated and immobilized in the hydrogel. As described above, different medicinal components can be slowly released from the gelatin hydrogel by chemically derivatizing or modifying the gelatin in such a way that it can interact with the medicinal component according to the chemical nature and physical properties of the medicinal component.

Although the process of cationization is not particularly limited as long as a method capable of introducing a functional group that is cationized under physiological conditions is used, a method of introducing a primary, secondary, or tertiary amino group or ammonium group into the hydroxyl group or carboxyl group of the gelatin under mild conditions is preferred. For example, a method of reacting alkyldiamine such as ethylenediamine, N,N-dimethyl-1,3-diaminopropane, trimethylammonium acetohydrazide, spermine, spermidine, diethylamide hydrochloride, or the like using various condensing agents such as 1-ethyl-3-(3-dimethylaminopropyl)carbodiimide hydrochloride, cyanuric chloride, N,N'-carbodiimidazole, cyanogen bromide, diepoxy compounds, tosyl chloride, dianhydride compounds such as diethyl triamine-N,N,N'N",N"-pentanoic dianhydride, trisyl chloride and the like is possible. Among them, a method of reacting ethylenediamine is preferred for its simplicity and versatility.

Because the sheet for corneal transplants containing the medicinal component attains the effect of sustained release of medicinal component and the effect of stabilizing medicinal component, it enables prolonged release of the medicinal component in the desired site in the controlled direction. As a result, the medicinal component effectively exerts its action in the lesion.

It is assumed that one million people worldwide and several tens of thousands people nationwide are eligible for corneal transplants, and among them, patients with bullous keratopathy caused by corneal endothelial damage account for about 80% of all the diseases that can be treated by corneal transplants. Because corneal endothelium does not regenerate once it is damaged, currently there is no effective therapy available except corneal transplants.

As shown above, the sheet for corneal transplants of the present invention can be produced by a simple method and has not only excellent operability, engraftment, and degradability, but also a sustained drug release effect as described above. The use of the patient-derived corneal endothelial cells can avoid the problem of rejection reaction and enables an attempt of rapid regeneration of the corneal endothelium. Further, since the gelatin hydrogel has a function of sustained release of the medicinal component, curing of disease can be further promoted by including physiologically active medicinal components into the gelatin hydrogel and allowing it to release the medicinal components for a necessary period of time.

### Examples

Hereinbelow, the present invention will be described in more detail with reference to Examples; however, the present invention is not limited to these Examples.

### Example 1: Preparation of the gelatin hydrogel sheet

Collagen was extracted from pig skin or cattle bone by alkali treatment (molecular weight: 98,000, isoelectric point: 5.0). Then, a 10 WT% aqueous solution of gelatin was adjusted. This solution was poured into a plastic petri dish and left for several days at room temperature to evaporate water, whereby a gelatin sheet was obtained. The gelatin sheet was then subjected to thermal dehydration treatment at 160°C and 0.01 Torr for 72 hours to chemically cross-link the gelatin molecules. The gelatin sheet thus obtained had a percentage water content of 97% and a thickness of 100 µm. It should be noted that thickness can be adjusted to 30 µm or more depending on the purpose.

### (1) Transparency

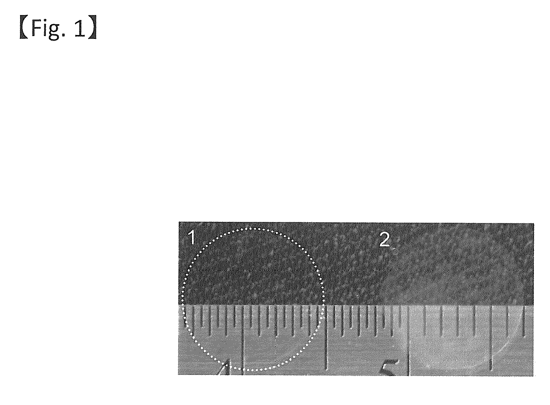

The gelatin hydrogel sheet prepared as above (48 hours of cross-linking) and an atelocollagen sheet (KOKEN CO., LTD.) were visually compared for transparency (Figure 1). Further, the light transmittance was compared among the gelatin hydrogel sheets subjected to various cross-linking times (6 to 48 hours). As shown in Table 1, at any cross-linking time, the light transmittance of the gelatin hydrogel was higher than that of atelocollagen.

**[Table 1]**

| | | Gelatin hydrogel (Dehydrothermal cross-linking time) | | | |
|---|---|---|---|---|---|
| | Atelocollagen | (6 hr) | (12 hr) | (24 hr) | (48 hr) |
| Transmittance (%) | 72.1±2.1 | 99.1±0.1 | 99.0±0.2 | 98.8±0.5 | 99.0±0.6 |

### (2) Mechanical characteristics

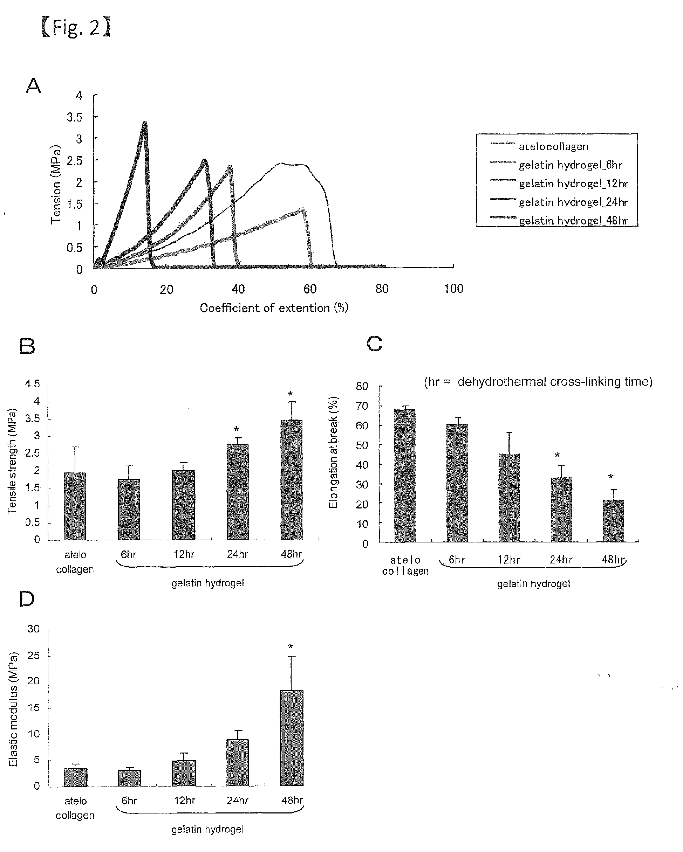

The mechanical characteristics of the gelatin hydrogel sheet and atelocollagen were compared and evaluated.
Strength in a tensile test (Figures 2A and B) and break points (Figure 2C) of the gelatin hydrogel sheets subjected to various cross-linking times (6 to 48 hours) and the atelocollagen sheet were measured and elastic moduli were calculated (Figure 2D).
The gelatin hydrogel sheet showed a tendency of increasing tensile strength with decreasing elastic modulus in a cross-linking time-dependent manner. Also, it was confirmed that the gelatin hydrogel sheet had equivalent or higher strength and elasticity compared to atelocollagen at any cross-linking time.

### (3) Substance permeability

The substance permeability of the gelatin hydrogel sheet (48 hours of cross-linking) and that of the atelocollagen sheet were compared and evaluated. The substance permeability was evaluated by obtaining diffusive coefficients of albumin and glucose by a routine method.

Compared to the atelocollagen sheet, the gelatin hydrogel sheet exhibited significantly higher permeation of albumin. Meanwhile, the gelatin hydrogel sheet and the atelocollagen sheet exhibited equivalent permeation of glucose. From these results, the gelatin hydrogel sheet was considered to be useful as a carrier sheet.

### Example 2: Cell culture on the gelatin hydrogel sheet

### 1. Preparation of corneal endothelial cells

Corneoscleral buttons were prepared from the rabbit eye and the Descemet's membrane was detached. The Descemet's membrane was incubated with 0.25% Trypsin-EDTA at 37°C for 10 minutes to isolate cells. To the cells, a DMEM medium (low glucose, Nikken Bio Medical Laboratory) containing 2 ng/ml bFGF (R&D systems, Inc.) and 10% serum (Japan bio serum) was added, followed by centrifugation at 300 g for five minutes. The supernatant was aspirated and the remaining cell aggregate was suspended in the same medium.

### 2. Study on the corneal endothelial cell culture and the coating conditions

The gelatin hydrogel sheet prepared in Example 1 was coated with type I or type IV collagen (Nitta Gelatin, Inc.). As the coating method, a method of directly applying a 3.0 mg/ml collagen stock solution (pH 3.0) on the gelatin hydrogel sheet prepared in Example 1 with a cell scraper and the like and a method of immersing the gelatin hydrogel sheet in a collagen solution diluted 10-fold with diluted hydrochloric acid were performed, and in both method, coating was formed by leaving the hydrogel sheet for 30 minutes or longer in a clean bench. The sheet thus coated was washed three times with Phosphate-Buffered Saline (PBS) (Invitrogen) before use.

Corneal endothelial cells isolated from the Descemet's membrane were seeded on this sheet at a seeding density of 1.5 to 4.5 × 10³ cells/mm² and cultured in a serum-containing endothelial culture medium used in the previous section at 37°C and 10% CO₂ for 14 days. As a comparison, corneal endothelial cells were seeded and cultured on atelocollagen under similar conditions.

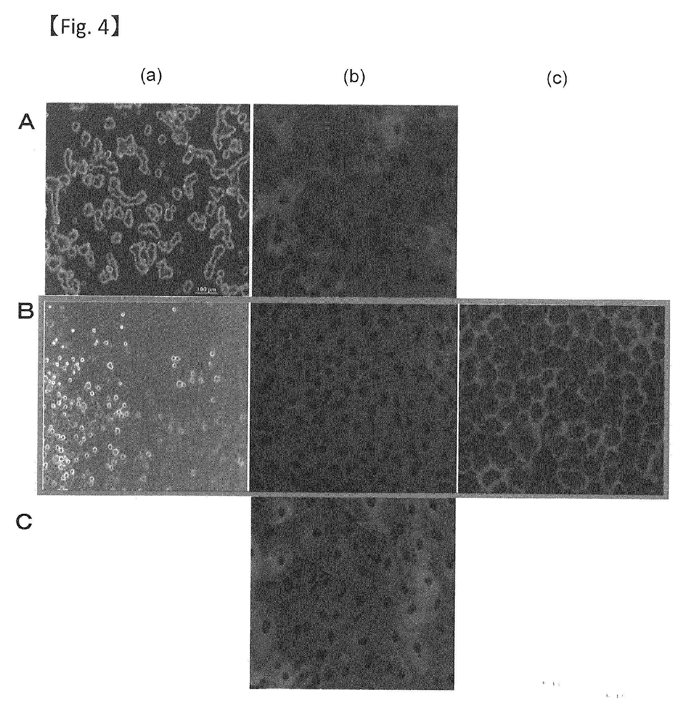

Cell adhesion to the gelatin hydrogel sheet directly coated with the collagen solution and to the gelatin hydrogel sheet immersed in the collagen solution was evaluated. Observation of the form of the endothelial cell under the phase-contrast microscopy on the day after seeding the cell revealed that most of the endothelial cells had adhered to the sheet coated with the stock solution of collagen solution, whereas apparently fewer cells had adhered to the sheet immersed in a 10-fold diluted collagen solution (Figures 4A-(a) and B-(a)). Also, cells after 10 days of culturing were observed under a phase-contrast microscope and subjected to immunofluorescent staining using an anti-ZO-1 antibody (Zymed) and an anti-Na⁺/K⁺-ATPase antibody. As a result, clear expression of the tight junction protein ZO-1 and the endothelial marker Na⁺/K⁺-ATPase was observed between the cells in the sheet coated with the stock solution of collagen (Figures 4B-(b) and (c)). Similarly to the gelatin sheet coated with the stock solution, clear ZO-1 expression was observed in the comparative atelocollagen sheet (Figure 4C-(b)). From these results, it was assumed that application of the stock solution of collagen to the gelatin hydrogel sheet would achieve similar adherence property and functional expression to those observed with atelocollagen.

### 3. Evaluation of the corneal endothelial cell sheet

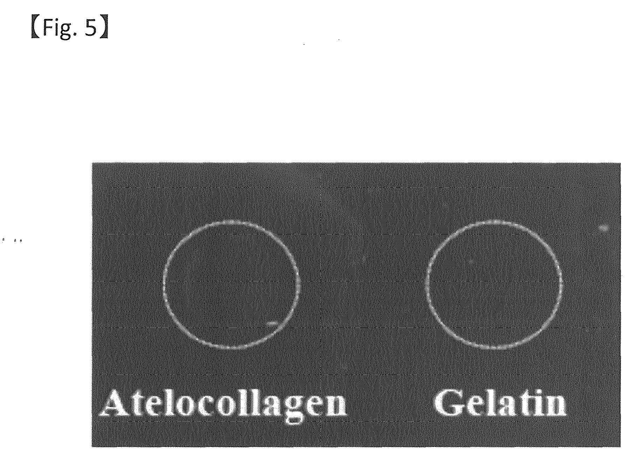

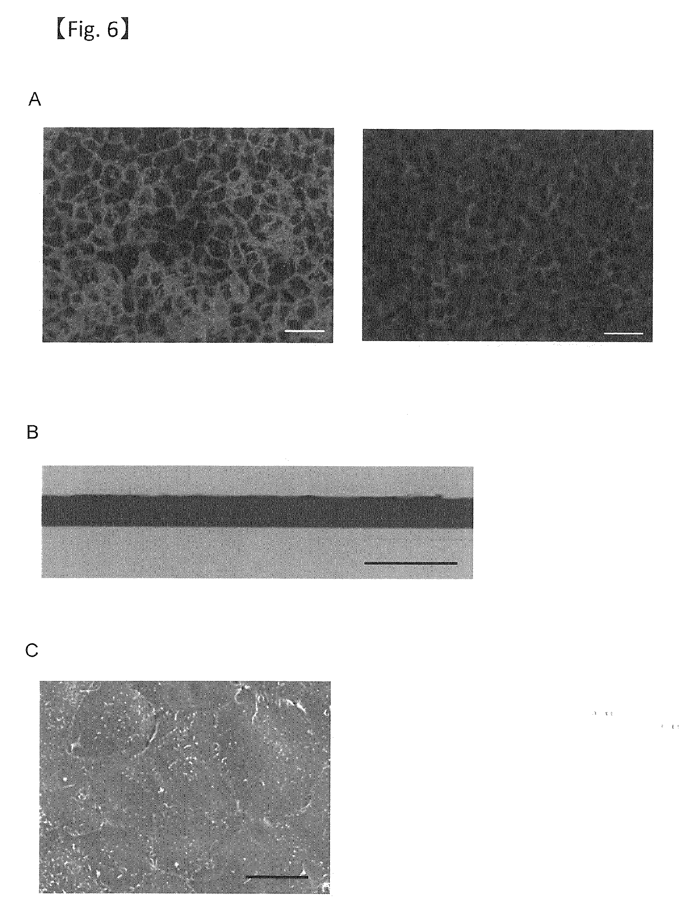

The corneal endothelial cell sheet obtained by culturing cells on the collagen-applied gelatin hydrogel as a carrier and the corneal endothelial cell sheet obtained by culturing cells on atelocollagen as a carrier were visually compared for transparency. While atelocollagen became cloudy and had reduced transparency after culturing, the transparency of the gelatin hydrogel sheet was not reduced by culturing (Figure 5).
Corneal endothelial cells cultured on the gelatin hydrogel exhibited the endothelial pump marker Na⁺/K⁺-ATPase (Figure 6A) and the tight junction marker ZO-1 (Figure 6B), Also, scanning microscopic observation revealed microvilli on the cell surface, which are the characteristics of the endothelial cell (Figure 6C). From the above results, it was shown that the gelatin hydrogel sheet supported the normal growth of corneal endothelial cells by serving as a scaffold.

### Example 3: Transplantation of the gelatin hydrogel sheet into the rabbit anterior chamber (study on biodegradability)

Following cataract extraction from the rabbit eye (phacoemulsification), the Descemet's membrane was detached. Then, a gelatin hydrogel sheet punched out with an 8 mm trepan was transplanted alone into the anterior chamber.

### (1) Observation image of the ocular surface

As of 28th day after transplantation, transparency of the sheet transplanted into the anterior chamber and transparency of the cornea were both maintained and the iris could be seen through. Also, there was no sign of inflammation, edema, or the like.

### (2) The gelatin hydrogel sheet after transplantation

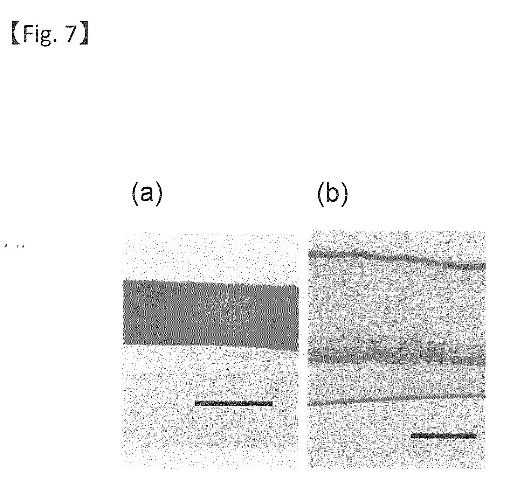

The rabbit was euthanized on postoperative day 28, and the eye ball was taken out and chemically fixed with a 10% neutral buffered formalin solution. Subsequently, the corneal tissue was observed with hematoxylin and eosin staining (Figure 7 (a) before transplantation, (b) after transplantation). The cornea tissue and the gelatin hydrogel sheet were adhered well and neither migration nor infiltration of inflammatory cells caused by the gelatin hydrogel was observed, and the cornea had good transparency. While the thickness of the gelatin hydrogel sheet before transplantation was about 192.7 ± 3.2 µm (N = 9), the thickness on the 28th day after transplantation was reduced to 163.8 µm (N = 9), exhibiting favorable degradability.

### Example 4: Transplantation of the cultured corneal endothelial cell sheet prepared with the gelatin hydrogel sheet into the rabbit model of bullous keratopathy

In accordance with the method of Example 2, rabbit-derived corneal endothelial cells were cultured on the gelatin hydrogel to prepare a cultured corneal endothelial cell sheet for transplants. The cultured corneal endothelial cell sheet thus prepared was transplanted into the rabbit model of bullous keratopathy from which the corneal endothelium had been completely removed. After transplantation, the corneal thickness was measured with a pachymeter and the anterior segment was observed.

### (1) Observation image of the ocular surface

On the 28th day after transplantation, transparency of the sheet transplanted into the anterior chamber and transparency of the cornea were both good and the iris could be seen through. Also, slit observation of the anterior segment showed no sign of inflammation, edema, or the like, and also, exfoliation of the sheet was not observed.

### (2) Corneal thickness of the transplanted eye, non-transplanted eye, and normal eye on the 7th and 21st days after transplantation

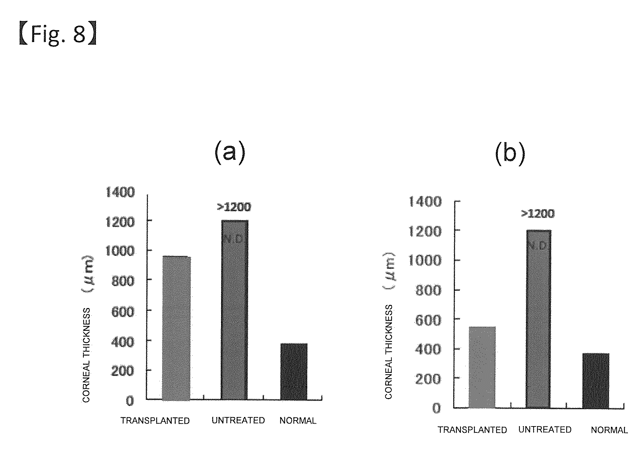

Corneal thickness was compared among the transplanted eye, non-transplanted eye, and normal eye on the 7th and 21st days after transplantation. As a result, cultured corneal endothelial cells transplanted using the gelatin sheet as a carrier were functional as of postoperative day 21 and exhibited improved corneal thickness and transparency (Figure 8).

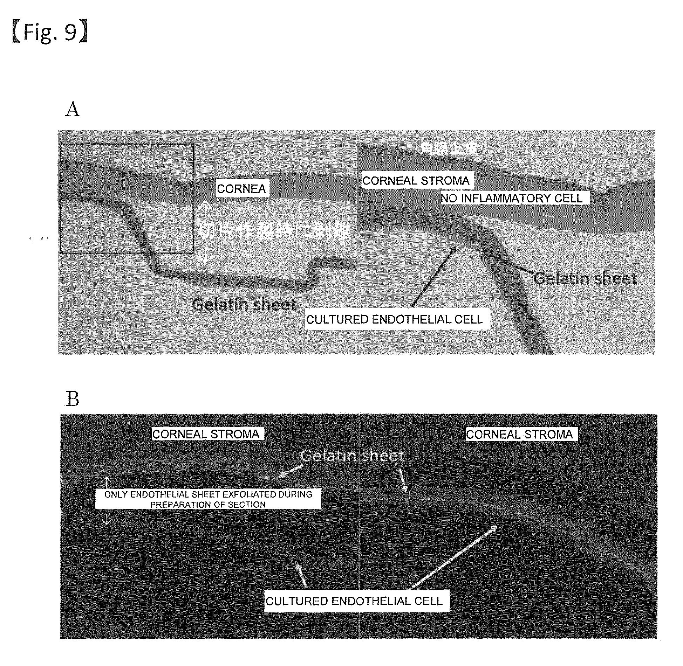

Separately, the eye ball was taken out on the 21st day after transplantation and subjected to HE staining (Figure 9A) and DiI staining (Figure 9B). In the HE staining, the gelatin sheet exfoliated from the stroma during preparation of a section; however, there was no accumulation of inflammatory cells at the adhesion site to the stroma (the image on the right is an enlarged view of the boxed part of the image on the left). DiI staining confirmed that DiI-positive, transplanted cultured endothelial cells remained on the gelatin sheet.

### Industrial Applicability

The sheet for corneal transplants having the gelatin hydrogel as a carrier of the present invention can be transplanted without impairing the function and form of endothelial cells. The sheet for corneal transplants of the present invention has favorable transparency and engraftment to the tissue after transplantation. Also, the rate of biodegradation in vivo can be adjusted by the preparation conditions, and it does not cause a problem such as inflammation reaction. Furthermore, the raw material gelatin hydrogel has already been clinically used. Accordingly, the sheet for corneal transplants of the present invention is extremely useful as an alternative therapy for corneal endothelial disease to conventional penetrating keratoplasty. Since the number of the patients with bullous keratopathy and the like eligible for a therapy with the sheet for corneal transplants of the present invention is estimated to reach several tens of thousands each year even in Japan alone, this therapy has a high potential commercial value.

All the publications, patents, and patent applications cited in the present specification are incorporated herein by reference in their entirety.

## Claims

1. A sheet for corneal transplants comprising corneal endothelial cells on a gelatin hydrogel.

2. The sheet for corneal transplants according to Claim. 1, wherein the thickness of the gelatin hydrogel is 10 to 200 µm.

3. The sheet for corneal transplants according to Claim 1 or 2, wherein the thickness of the gelatin hydrogel is 30 to 100 µm.

4. The sheet for corneal transplants according to any one of Claims 1 to 3, wherein the gelatin hydrogel has a percentage water content of 92 to 99%.

5. The sheet for corneal transplants according to any one of Claims 1 to 4, wherein the density of cells on the gelatin hydrogel is 2000 cells/mm² or more.

6. The sheet for corneal transplants according to any one of Claims 1 to 5, wherein the sheet for corneal transplants comprises ZO-1-positive corneal endothelial cells.

7. The sheet for corneal transplants according to any one of Claims 1 to 6, wherein the gelatin hydrogel is prepared by subjecting gelatin to thermal cross-linking treatment.

8. The sheet for corneal transplants according to any one of Claims 1 to 7, wherein the gelatin hydrogel is degraded in vivo after transplantation.

9. The sheet for corneal transplants according to any one of Claims 1 to 8, wherein the gelatin hydrogel is degraded in vivo and disappears within one to six months after transplantation.

10. The sheet for corneal transplants according to any one of Claims 1 to 8, wherein the gelatin hydrogel is engrafted into a stromal tissue without being biodegraded.

11. The sheet for corneal transplants according to any one of Claims 1 to 10, wherein the corneal endothelial cells are patient-derived cells.

12. The sheet for corneal transplants according to any one of Claims 1 to 11, wherein the sheet for corneal transplants is obtainable by seeding and culturing corneal endothelial cells on a gelatin hydrogel sheet coated with collagen.

13. A method for producing a sheet for corneal transplants, comprising seeding and culturing corneal endothelial cells on a gelatin hydrogel sheet coated with collagen.

14. The method according to Claim 13, wherein the method comprises the step of applying collagen over a surface of the gelatin hydrogel sheet.

15. The method according to Claim 13 or 14, wherein the method comprises detaching the gelatin hydrogel sheet with the corneal endothelial cells from a culture container on or after the seventh day after the corneal endothelial cells become confluent.

16. The method according to any one of Claims 13 to 15, wherein the density of cells on the gelatin hydrogel in the obtained sheet for corneal transplants is 2000 cells/mm² or more.
